Europäisches Patentamt

(19) European Patent Office

Office européen des brevets

(11) Publication number: **0 361 659 B1**

# EUROPEAN PATENT SPECIFICATION

(12)

(45) Date of publication of patent specification :
**18.12.91 Bulletin 91/51**

(51) Int. Cl.⁵ : **A61K 31/47, A61K 9/72**

(21) Application number : **89308104.2**

(22) Date of filing : **09.08.89**

(54) Pharmaceutical composition.

(30) Priority : **26.08.88 GB 8820267**

(43) Date of publication of application :
**04.04.90 Bulletin 90/14**

(45) Publication of the grant of the patent :
**18.12.91 Bulletin 91/51**

(84) Designated Contracting States :
**AT BE CH DE ES FR GB GR IT LI LU NL SE**

(56) References cited :
**EP-A- 0 285 246**
**GB-A- 2 022 078**
**GB-A- 2 157 291**
**US-A- 3 957 965**

(73) Proprietor : **FISONS plc**
**Fison House Princes Street**
**Ipswich Suffolk IP1 1QH (GB)**

(72) Inventor : **Clark, Andrew Reginald**
**32 Braddon Road**
**Loughborough Leicestershire (GB)**
Inventor : **Wright, Paul**
**7 Thornhill Close**
**Bramcote Nottingham (GB)**

(74) Representative : **Craig, Christopher Bradberry**
**et al**
**Fisons plc 12 Derby Road**
**Loughborough Leicestershire LE11 0BB (GB)**

## Description

This invention relates to novel pharmaceutical formulations, processes for their preparation.

The administration of drugs by inhalation to the nose or lungs is well-known and drugs to be administered by this route are commonly formulated as powders, either as pressurised aerosols or as dry powders of fine particle size in admixture with coarser particles of an inert carrier. One drug which is known, eg from US Patent No 4760072, to be effective in the treatment of inter alia reversible obstructive airways disease when administered by inhalation is nedocromil sodium, the disodium salt of 9-ethyl-6,9-dihydro-4,6-dioxo-10-propyl-4H-pyrano[3,2-g]quinoline-2,8-dicarboxylic acid. However, the use of this drug but is not without certain disadvantages. For example, whilst the duration of action of nedocromil sodium is considerable, it is sometimes insufficient to enable a patient to obtain a full night's sleep. Also, some patients experience side effects with nedocromil sodium, including a feeling of warmth or an undesirable taste.

We have now surprisingly found that nedocromil calcium, the calcium salt of 9-ethyl-6,9-dihydro-4,6-dioxo-10-propyl-4H-pyrano[3,2-g]quinoline-2,8-dicarboxylic acid, in finely divided form containing approximately 10% w/w water possesses advantageous properties when formulated in admixture with a coarser carrier.

Thus, according to the invention, there is provided a pharmaceutical composition comprising particles of nedocromil calcium having a mass median diameter of from 0.01 to 10 μm (microns) and a water content of from 6 to 12% w/w in admixture with coarser particles of a solid pharmaceutically acceptable carrier.

The composition according to the invention is useful in the inhalation treatment of the conditions known by the generic term 'reversible obstructive airways disease'. The composition may, however, also be administered by nasal insufflation, or orally, eg for the treatment of certain inflammatory conditions of the gastro-intestinal tract.

The composition according to the invention is advantageous in that when administered to a patient by inhalation, the 'dispersion', ie the proportion of the particles of the active ingredient which are fine enough to penetrate deep into the lung, is greater than is the case with similar formulations of other drugs. This leads to a greater effective dose administered to the patient or, conversely, to the possibility of achieving the same effective dose from a smaller nominal dose. Also, the dispersion may be less dependent on the flow rate of air through the device from which the drug is dispensed, leading to a more uniform dose. In addition, there may be further advantages such as fewer or reduced side effects and/or greater duration of action.

The composition according to the invention may also exhibit certain technical advantages. For example, the emptying properties of the compositions from capsules may be superior to those of similar formulations of other drugs. Also, the stability of the composition may be greater, particularly when the compositions are put up in capsules, eg gelatine capsules.

The composition preferably comprises between 20 and 80% w/w of nedocromil calcium, more preferably between 40 and 60%, for example about 50%, and from 80 to 20%, more preferably from 60 to 40%, for example about 50% w/w of the carrier.

The nedocromil calcium particles preferably have a mass median diameter in the range 1 to 10 μm (microns), more preferably 2 to 6 μm (microns), eg about 3 to 4 μm (microns). Preferably at least 50% by weight of the nedocromil calcium particles have particle sizes in the range 2 to 6 μm (microns).

The solid pharmaceutically acceptable carrier in the composition will generally be a non-toxic material chemically inert to nedocromil calcium but may, if so desired, also comprise larger particles of nedocromil calcium. Examples of carriers which may be used in the composition include a dextran, mannitol and, preferably, lactose. A particularly preferred carrier is crystalline lactose.

The particles of carrier preferably have a mass median diameter of from about 30 to 150 μm (microns). The mass median diameter is preferably less than 100 μm (microns) and more preferably less than 80 μm (microns). It is particularly preferred that the mass median diameter of the carrier particles be in the range 30 to 80 μm (microns), eg about 50 to 60 μm (microns).

It is especially preferred that the composition be substantially free of particles having particle sizes in the range 11 to 29 μm (microns). The term 'substantially free' is used to indicate that the composition contains less than 10%, more preferably less than 5% w/w of particles having particle sizes in the range 11 to 29 μm (microns).

The nedocromil calcium preferably has a water content of between 8 and 12% w/w, more preferably between 10 and 12%, and especially between 10 and 11%.

According to a particularly preferred aspect of the invention there is provided a pharmaceutical composition comprising particles of nedocromil calcium having a mass median diameter of from 2 to 6 μm (microns) and a water content of from 8 to 12% w/w, in admixture with a solid pharmaceutically acceptable carrier having a mass median diameter of from 30 to 80 μm (microns), the proportion by weight of nedocromil calcium to carrier being in the range 1 : 4 to 4 : 1.

The finely divided nedocromil calcium may be prepared by direct milling down to the desired particle size range. The particulate carrier may be prepared by grinding the carrier and subsequently separating out the desired fraction by conventional methods, eg by air classification and sieving.

The composition may be prepared by mixing the ingredients together in a mixer, eg a planetary or other stirred mixer. The invention thus also provides a method for preparing a composition of the invention which comprises mixing together the finely divided nedocromil calcium and the coarse carrier, after comminution and particle size classification of the ingredients if this is necessary.

The compositions according to the invention will generally be put up in gelatine, plastics or other capsules.

The amount of composition contained in the capsule will, of course, to some extent depend on the desired dosage, but will generally be from about 10 to 100 mg, eg 20 mg, 30 mg or 40 mg.

There is also provided therefore, as a further feature of the invention, a dosage unit comprising a capsule containing from 10 to 100 mg of a pharmaceutical composition comprising particles of nedocromil calcium having a mass median diameter of from 0.01 to 10 $\mu$m (microns) in admixture with coarser particles of a solid pharmaceutically acceptable carrier, wherein the nedocromil calcium has a water content of from 6 to 12% w/w.

Nedocromil calcium having a water content in the range 6 to 12% w/w may be prepared by the method described in Example 1, and typical compositions of the invention are illustrated in Example 2.

## Example 1

### Calcium 9-ethyl-6,9-dihydro-4,6-dioxo-10-propyl-4H-pyrano[3,2-g]quinoline-2,8-dicarboxylate

A filtered solution of disodium 9-ethyl-6,9-dihydro-4,6-dioxo-10-propyl-4H-pyrano[3,2-g]quinoline-2,8-dicarboxylate (500 g) in 3.4 litres of water was treated with a filtered solution of calcium nitrate tetrahydrate (513 g) in 3.4 litres of acetone. The mixture was stirred slowly for 2 hours at ambient temperature and the precipitate then filtered off and washed with water and acetone. The solid was then slurried in 3 litres of water and filtered ; this was followed by a slurry in 3-4 litres of acetone and refiltration.

The damp solid was dried, in vacuo, at 65°C to give 547 g of the title compound (contains 10.9% w/w water).

$C_{19}H_{15}NO_7Ca$

| | |
|---|---|
| Requires : | C,55.74 ; H,3.69 ; N,3.42 ; Ca,9.79% |
| Requires : | C,49.65 ; H,4.50 ; N,3.05 ; Ca,8.72% (for 10.9M water) |
| Found : | C,49.65 ; H,4.51 ; N,3.17, Ca,7.93% |

## Example 2

| | Ingredients | % w/w |
|---|---|---|
| a) | Nedocromil calcium | 50 |
| | Crystalline lactose | 50 |
| b) | Nedocromil calcium | 30 |
| | Crystalline lactose | 70 |
| c) | Nedocromil calcium | 70 |
| | Crystalline lactose | 30 |

In each case the nedocromil calcium has a water content of 10-11% w/w and is micronised to a mass median diameter of approximately 4 $\mu$m (microns). The crystalline lactose is classified and has a mass median diameter of approximately 55 $\mu$m (microns). The compositions are prepared by mixing the ingredients in a planetary mixer.

## Claims

**Claims for the following Contracting States : AT, BE, CH, DE, FR, GB, IT, LI, LU, NL**

1. A pharmaceutical composition comprising particles of nedocromil calcium having a mass median diameter of from 0.01 to 10 µm (microns) and a water content of from 6 to 12% w/w in admixture with coarser particles of a solid pharmaceutically acceptable carrier.

2. A composition according to Claim 1, which comprises between 20 and 80% w/w of nedocromil calcium.

3. A composition according to Claim 1 or Claim 2, wherein the nedocromil calcium particles have a mass median diameter in the range 2 to 6 µm (microns).

4. A composition according to any one of the preceding claims, wherein the carrier is crystalline lactose.

5. A composition according to any one of the preceding claims, wherein the particles of carrier have a mass median diameter of from 30 to 80 µm (microns).

6. A composition according to any one of the preceding claims which is substantially free of particles having particle sizes in the range 11 to 29 µm (microns).

7. A composition according to any one of the preceding claims, wherein the nedocromil calcium has a water content of between 8 and 12% w/w.

8. A pharmaceutical composition comprising particles of nedocromil calcium having a mass median diameter of from 2 to 6 µm (microns) and a water content of from 8 to 12% w/w, in admixture with a solid pharmaceutically acceptable carrier having a mass median diameter of from 30 to 80 µm (microns), the proportion by weight of nedocromil calcium to carrier being in the range 1 : 4 to 4 : 1.

9. A dosage unit comprising a capsule containing from 10 to 100 mg of a pharmaceutical composition comprising particles of nedocromil calcium having a mass median diameter of from 0.01 to 10 µm (microns) and a water content of from 6 to 12% w/w in admixture with coarser particles of a solid pharmaceutically acceptable carrier.

**Claims for the following Contracting States : ES, GR**

1. A process for the preparation of a pharmaceutical composition comprising particles of nedocromil calcium having a mass median diameter of from 0.01 to 10 µm (microns) and a water content of from 6 to 12% w/w in admixture with coarser particles of a solid pharmaceutically acceptable carrier, which process comprises mixing the ingredients.

2. A process according to Claim 1, wherein the composition comprises between 20 and 80% w/w of nedocromil calcium.

3. A process according to Claim 1 or Claim 2, wherein the nedocromil calcium particles have a mass median diameter in the range 2 to 6 µm (microns).

4. A process according to any one of the preceding claims, wherein the carrier is crystalline lactose.

5. A process according to any one of the preceding claims, wherein the particles of carrier have a mass median diameter of from 30 to 80 µm (microns).

6. A process according to any one of the preceding claims, wherein the composition is substantially free of particles having particle sizes in the range 11 to 29 µm (microns).

7. A process according to any one of the preceding claims, wherein the nedocromil calcium has a water content of between 8 and 12% w/w.

8. A process for the preparation of a pharmaceutical composition comprising particles of nedocromil calcium having a mass median diameter of from 2 to 6 µm (microns) and a water content of from 8 to 12% w/w, in admixture with a solid pharmaceutically acceptable carrier having a mass median diameter of from 30 to 80 µm (microns), the proportion by weight of nedocromil calcium to carrier being in the range 1 : 4 to 4 : 1, which process comprises mixing the ingredients.

9. A process for the preparation of a dosage unit comprising a capsule containing a pharmaceutical composition comprising particles of nedocromil calcium having a mass median diameter of from 0.01 to 10 µm (microns) and a water content of from 6 to 12% w/w in admixture with coarser particles of a solid pharmaceutically acceptable carrier, which process comprises filling a capsule with from 10 to 100 mg of the composition.

## Patentansprüche

## Patentansprüche für folgende Vertragsstaaten : AT, BE, CH, DE, FR, GB, IT, LI, LU, NL, SE

1. Pharmazeutische Zusammensetzung, umfassend Kalziumnedocromilteilchen mit einem gehäuften Mediandurchmesser von 0,01 bis 10 μm (Mikron) und einem Wassergehalt von 6 bis 12% G/G in Mischung mit gröberen Teilchen eines festen, pharmazeutisch annehmbaren Trägers.

2. Zusammensetzung nach Anspruch 1, umfassend zwischen 20 und 80% G/G Kalziumnedocromil.

3. Zusammensetzung nach Anspruch 1 oder 2, wobei die Kalziumnedocromilteilchen einen gehäuften Mediandurchmesser im Bereich von 2 bis 6 μm (Mikron) aufweisen.

4. Zusammensetzung nach einem der vorhergehenden Ansprüche, wobei der Träger kristalline Lactose ist.

5. Zusammensetzung nach einem der vorhergehenden Ansprüche, wobei die Teilchen des Trägers einen gehäuften Mediandurchmesser von 30 bis 80 μm (Mikron) aufweisen.

6. Zusammensetzung nach einem der vorhergehenden Ansprüche, die von Teilchen mit Teilchengrößen im Bereich von 11 bis 29 μm (Mikron) im wesentlichen frei ist.

7. Zusammensetzung nach einem der vorhergehenden Ansprüche, wobei das Kalziumnedocromil einen Wassergehalt von zwischen 8 und 12% G/G aufweist.

8. Pharmazeutische Zusammensetzung, umfassend Kalziumnedocromilteilchen mit einem gehäuften Mediandurchmesser von 2 bis 6 μm (Mikron) und einem Wassergehalt von 8 bis 12% G/G in Mischung mit einem festen, pharmazeutisch annehmbaren Träger mit einem gehäuften Mediandurchmesser von 30 bis 80 μm (Mikron), wobei das Gewichtsverhältnis von Kalziumnedocromil zu Träger im Bereich von 1 : 4 bis 4 : 1 liegt.

9. Dosierungseinheit, umfassend eine Kapsel enthaltend 10 bis 100 mg einer pharmazeutischen Zusammensetzung, umfassend Kalziumnedocromilteilchen mit einem gehäuften Mediandurchmesser von 0,01 bis 10 μm (Mikron) und einem Wassergehalt von 6 bis 12% G/G in Mischung mit gröberen Teilchen eines festen, pharmazeutisch annehmbaren Trägers.

## Patentansprüche für folgende Vertragsstaaten : ES, GR

1. Verfahren zur Herstellung einer pharmazeutischen Zusammensetzung, umfassend Kalziumnedocromilteilchen mit einem gehäuften Mediandurchmesser von 0,01 bis 10 μm (Mikron) und einem Wassergehalt von 6 bis 12% G/G in Mischung mit gröberen Teilchen eines festen, pharmazeutisch annehmbaren Trägers, welches Verfahren das Mischen der Inhaltsstoffe umfaßt.

2. Verfahren nach Anspruch 1, wobei die Zusammensetzung zwischen 20 und 80% G/G Kalziumnedocromil umfaßt.

3. Verfahren nach Anspruch 1 oder 2, wobei die Kalziumnedocromilteilchen einen gehäuften Mediandurchmesser im Bereich von 2 bis 6 μm (Mikron) aufweisen.

4. Verfahren nach einem der vorhergehenden Ansprüche, wobei der Träger kristalline Lactose ist.

5. Verfahren nach einem der vorhergehenden Ansprüche, wobei die Teilchen des Trägers einen gehäuften Mediandurchmesser von 30 bis 80 μm (Mikron) aufweisen.

6. Verfahren nach einem der vorhergehenden Ansprüche, wobei die Zusammensetzung von Teilchen mit Teilchengrößen im Bereich von 11 bis 29 μm (Mikron) im wesentlichen frei ist.

7. Verfahren nach einem der vorhergehenden Ansprüche, wobei das Kalziumnedocromil einen Wassergehalt von zwischen 8 und 12% G/G aufweist.

8. Verfahren zur Herstellung einer pharmazeutischen Zusammensetzung, umfassend Kalziumnedocromilteilchen mit einem gehäuften Mediandurchmesser von 2 bis 6 μm (Mikron) und einem Wassergehalt von 8 bis 12% G/G in Mischung mit einem festen, pharmazeutisch annehmbaren Träger mit einem gehäuften Mediandurchmesser von 30 bis 80 μm (Mikron), wobei das Gewichtsverhältnis von Kalziumnedocromil zu Träger im Bereich von 1 : 4 bis 4 : 1 liegt, welches Verfahren das Mischen der Inhaltsstoffe umfaßt.

9. Verfahren zur Herstellung einer Dosierungseinheit, umfassend eine Kapsel enthaltend eine pharmazeutische Zusammensetzung, umfassend Kalziumnedocromilteilchen mit einem gehäuften Mediandurchmesser von 0,01 bis 10 μm (Mikron) und einem Wassergehalt von 6 bis 12% G/G in Mischung mit gröberen Teilchen eines festen, pharmazeutisch annehmbaren Trägers, welches Verfahren das Füllen einer Kapsel mit 10 bis 100 mg der Zusammensetzung umfaßt.

**Revendications**

**Revendications pour les Etats contractants suivants : AT, BE, CH, DE, FR, GB, IT, LI, LU, NL, SE**

1. Composition pharmaceutique comprenant des particules de nédocromil calcique ayant un diamètre médian en masse de 0,01 à 10 µm (microns) et une teneur en eau de 6 à 12% p/p en mélange avec des particules plus grossières d'un excipient solide pharmaceutiquement acceptable.

2. Composition suivant la revendication 1, qui comprend entre 20 et 80% p/p de nédocromil calcique.

3. Composition suivant la revendication 1 ou 2, dans laquelle le nédocromil calcique a un diamètre médian en masse de l'intervalle de 2 à 6 µm (microns).

4. Composition suivant l'une quelconque des revendications précédentes, dans laquelle l'excipient est le lactose cristallin.

5. Composition suivant l'une quelconque des revendications précédentes, dans laquelle les particules de l'excipient ont un diamètre médian en masse de 30 à 80 µm (microns).

6. Composition suivant l'une quelconque des revendications précédentes, qui est sensiblement exempte de particules ayant des granulométries dans l'intervalle de 11 à 29 µm (microns).

7. Composition suivant l'une quelconque des revendications précédentes, dans laquelle le nédocromil calcique a une teneur en eau entre 8 et 12% p/p.

8. Composition pharmaceutique comprenant des particules de nédocromil calcique ayant un diamètre médian en masse de 2 à 6 µm (microns) et une teneur en eau de 8 à 12% p/p, en mélange avec un excipient solide pharmaceutiquement acceptable ayant un diamètre médian en masse de 30 à 80 µm (microns), la proportion en poids du nédocromil calcique à l'excipient se situant dans l'intervalle de 1 : 4 à 4 : 1.

9. Unité dosée comprenant une capsule contenant 10 à 100 mg d'une composition pharmaceutique comprenant des particules de nédocromil calcique ayant un diamètre médian en masse de 0,01 à 10 µm (microns) et une teneur en eau de 6 à 12% p/p, en mélange avec des particules plus grossières d'un excipient solide pharmaceutiquement acceptable.

**Revendications pour les Etats contractants suivants : ES, GR**

1. Procédé de préparation d'une composition pharmaceutique comprenant des particules de nédocromil calcique ayant un diamètre médian en masse de 0,01 à 10 µm (microns) et une teneur en eau de 6 à 12% p/p en mélange avec des particules plus grossières d'un excipient solide pharmaceutiquement acceptable, lequel procédé comprend l'action de mélanger les constituants.

2. Procédé suivant la revendication 1, dans lequel la composition comprend entre 20 et 80% p/p de nédocromil calcique.

3. Procédé suivant la revendication 1 ou 2, dans lequel le nédocromil calcique a un diamètre médian en masse de l'intervalle de 2 à 6 µm (microns).

4. Procédé suivant l'une quelconque des revendications précédentes, dans lequel l'excipient est le lactose cristallin.

5. Procédé suivant l'une quelconque des revendications précédentes, dans lequel les particules de l'excipient ont un diamètre médian en masse de 30 à 80 µm (microns).

6. Procédé suivant l'une quelconque des revendications précédentes, dans lequel la composition est sensiblement exempte de particules ayant des granulométries dans l'intervalle de 11 à 29 µm (microns).

7. Procédé suivant l'une quelconque des revendications précédentes, dans lequel le nédocromil calcique a une teneur en eau entre 8 et 12% p/p.

8. Procédé de préparation d'une composition pharmaceutique comprenant des particules de nédocromil calcique ayant un diamètre médian en masse de 2 à 6 µm (microns) et une teneur en eau de 8 à 12% p/p, en mélange avec un excipient solide pharmaceutiquement acceptable ayant un diamètre médian en masse de 30 à 80 µm (microns), la proportion en poids du nédocromil calcique à l'excipient se situant dans l'intervalle de 1: 4 à 4 : 1, lequel procédé comprend l'action de mélanger les constituants.

9. Procédé de préparation d'une unité dosée comprenant une capsule contenant une composition pharmaceutique comprenant des particules de nédocromil calcique ayant un diamètre médian en masse de 0,01 à 10 µm (microns) et une teneur en eau de 6 à 12% p/p, en mélange avec des particules plus grossières d'un excipient solide pharmaceutiquement acceptable, lequel procédé comprend le remplissage d'une capsule avec 10 à 100 mg de la composition.